(19) Europäisches Patentamt
European Patent Office
Office européen des brevets

(11) Publication number: **0 106 962 A1**

# (12) EUROPEAN PATENT APPLICATION

(21) Application number: **83108038.7**

(51) Int. Cl.³: **C 07 D 233/64**

(22) Date of filing: **13.08.83**

(30) Priority: **24.09.82 US 422615**

(43) Date of publication of application: **02.05.84**
**Bulletin 84/18**

(84) Designated Contracting States: **AT BE CH DE FR GB IT LI LU NL SE**

(71) Applicant: **AMERICAN CYANAMID COMPANY,**
**1937 West Main Street P.O. Box 60, Stamford Connecticut 06904 (US)**

(72) Inventor: **Padmanathan, Thurairajah, 1 Devon Drive West, Piscataway New Jersey (US)**

(74) Representative: **Wächtershäuser, Günter, Dr., Tal 29, D-8000 München 2 (DE)**

**(54) Process for preparing 4(or 5)-(1-hydroxyalkyl) imidazoles.**

(57) A process is disclosed for preparing a compound of the formula

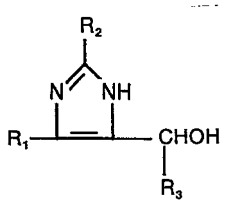

(I)

wherein $R_1$ and $R_3$ are the same or a different straight or branched chain lower alkyl group; and $R_2$ is hydrogen, a straight or branched chain lower alkyl, benzyl, or phenyl group, or a phenyl group substituted with a halo, a straight or branched chain lower alkyl or alkoxy, an amino, a lower dialkylamino or a nitro group

The process comprises adding a compound of the formula

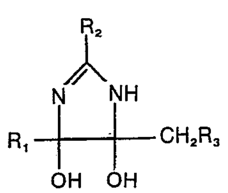

(II)

to an aqueous solution of an acid to form a reaction mixture, the mole ratio of the acid to formula (II) being about 1.75 to 2.5; maintaining the reaction mixture at a temperature of about 85 to 95°C for about 1 to 3 hours; neutralizing the reaction mixture; recovering the precipitate effected by the neutralizing step; and separating from the precipitate the compound of formula (I).

The compounds 4 (or 5)-methyl-5 (or 4)-ethyl-2-propyl-2-imidazoline-4, 5-diol hydrochloride (or 4), and 4 (or 5)-methyl-2,5 (or 2,4)-di-n-propyl-2-imidazoline-4,5-diol are also disclosed.

EP 0 106 962 A1

PROCESS FOR PREPARING 4(OR 5)-(1-HYDROXYALKYL)IMIDAZOLES

This invention relates to 4-(1-hydroxyalkyl) imidazoles, more particularly 5(or4)-alkyl-4(or 5)-(-1-hydroxyalkyl)imidazoles.

A process is provided for preparing a compound of the formula

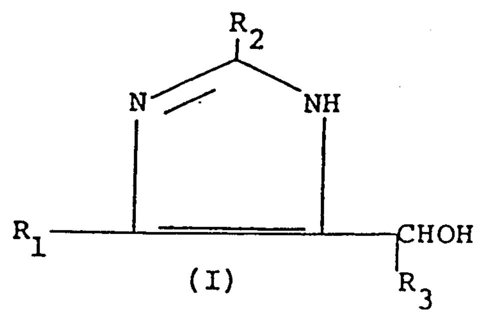

(I)

wherein $R_1$ and $R_3$ are the same or a different straight or branched chain lower alkyl group; and $R_2$ is hydrogen, a straight or branched chain lower alkyl, benzyl, or phenyl group, or a phenyl group substituted with a halo, a straight or branched chain lower alkyl or alkoxy, an amino, a lower dialkylamino or a nitro group.

The compounds of formula (I) can be oxidized to form the corresponding 4-acyl-imidazoles which are intermediate compounds for the preparation of imidazo[1,5-d]-AS--triazine-4-(3H)-ones and -thiones. The imidazo[1,5-d]-AS--triazine-4-(3H)-ones and -thiones are useful as broad spectrum herbicides and for the treatment of psoriasis.

A process for preparing a compound of the formula

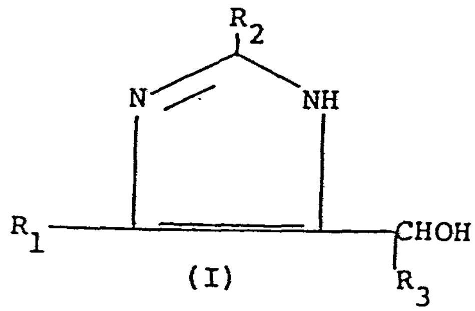

(I)

wherein $R_1$ and $R_3$ are the same or a different straight or branched chain lower alkyl group; and $R_2$ is hydrogen, a straight or branched chain lower alkyl, benzyl, or phenyl group, or a phenyl group substituted with a halo, a straight or branched chain lower alkyl or alkoxy, an amino, a lower dialkylamino or a nitro group has been invented. The process comprises:

adding a compound of the formula

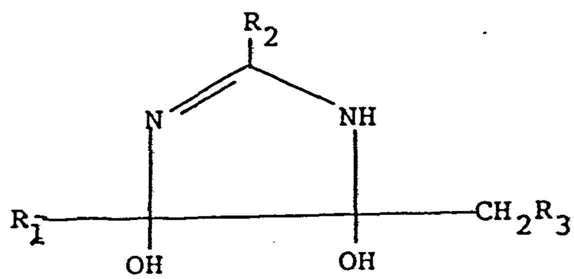

(II)

to an aqueous solution of an acid to form a reaction mixture, the mole ratio of said acid to said formula (II) being about 1.75 to 2.5;

maintaining said reaction mixture at a temperature of about 85 to 95°C for about 1 to 3 hours;

neutralizing said reaction mixture;

recovering the precipitate effected by said neutralizing step; and

separating from said precipitate the compound of formula (I).

It is to be understood that the compounds represented by formulas (I) and (II) are tautomers. That is, they are in equilibrium between the two isomeric structures (a) and (b)

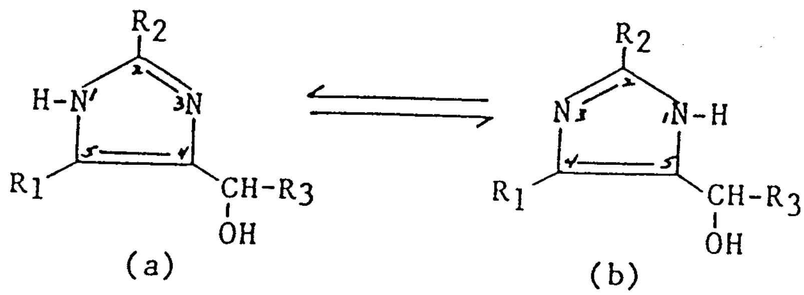

(I)

and

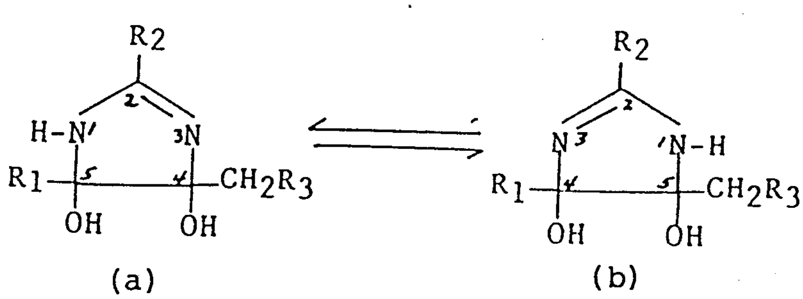

(II)

It is further to be understood that the compounds undergo the same reactions in either form. Finally, it is to be understood that the tautomerism is implicit in any compound represented by formulas (I) and (II).

Other embodiments of this invention are wherein $R_1$ and $R_3$ are methyl, and $R_2$ is n-propyl; wherein $R_1$ and $R_3$ are methyl, and $R_2$ is phenyl; and wherein $R_1$ is methyl, $R_2$ is propyl and $R_3$ is ethyl.

Still other embodiments are wherein said acid is selected from the group consisting of hydrochloric, sulfuric, phosphoric, boric and p-toluenesulfonic. In a specific embodiment, said acid is hydrochloric acid.

Yet other embodiments are wherein the mole ratio of said acid to said formula (II) is about 1.9 to 2.2; and wherein said reaction mixture is maintained at about 90 to 92°C for about one hour.

The following compounds have also been invented: 4 (or 5)-methyl-5 (or 4)-ethyl-2-propyl-2-imidazoline-4,5-diol hydrochloride (or 4); and 4 (or 5)-methyl-2,5 (or 2,4)-di-n-propyl-2-imidazoline-4,5-diol.

In the prior art, compounds of formula (I) can be prepared by dehydrating a compound of formula (III)

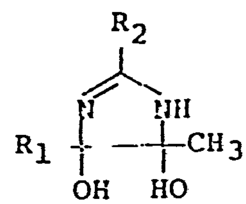

(III)

and then rearranging the dehydrated material to form a compound of formula (IV)

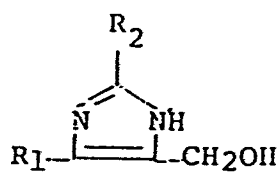

(IV).

The latter compound is oxidized to form the carboxaldehyde (V)

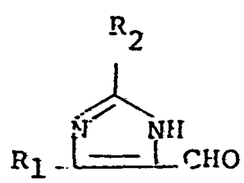

(V)

which is then reacted with an alkylmagnesium halide in a Grignard reaction to form the corresponding secondary alcohol of formula (I).

The preparation of 4(or 5)-(1-hydroxyethyl)-5(or 4)-methyl-2-phenylimidazole by the reaction of benzamidine hydrochloride and 3,4-epoxy-4-methyl-2-pentanone is described.

The process of the present invention offers the following advantages over the prior art:

1. It eliminates the need of compounds of formula (V), thus eliminating the need to oxidize compounds of formula (IV). Since this oxidation is usually carried out with nitric acid, the present process eliminates a step which can be hazardous.

2. The elimination of the carboxaldehydes of formula (V) also avoids the need for the formation of compounds of formula (I) by means of a Grignard reaction, which also can be hazardous.

## Description

In carrying out the process of this invention, a mixture of a suitable acid and an acid salt of a diol of formula (II) in water is prepared. The amounts used are such as to provide a mole ratio of acid to mixed diol salt of about 1.75 to 2.5, preferably about 1.9 to 2.2.

Suitable acids, which may be used in this mixture, include hydrochloric, sulfuric, phosphoric, boric, p-toluenesulfonic, and the like.

Suitable acid salts of 2-imidazoline-4,5-diols include the hydrochlorides, sulfates, phosphates, borates, p-toluenesulfonates, and the like, of the following:

4(or 5)-ethyl-5(or 4)-methyl-2-propyl-2-imidazoline-4,5-diol,

4,5-diethyl-2-propyl-2-imidazoline-4,5-diol,

4,5-diethyl-2-imidazoline-4,5-diol,

4(or 5)-ethyl-5(or 4)-methyl-2-phenyl-2-imidazoline-4,5-diol,

4,5-diethyl-2-(4-bromophenyl)-2-imidazoline-4,5-diol,

4,5-di-n-propyl-2-(4-nitrophenyl)-2-imidazoline-4,5-diol,

4(or 5)-ethyl-5(or 4)-methyl-2-benzyl-2-imidazoline-4,5-diol,

4(or 5)-ethyl-5(or 4)-methyl-2-(4-dimethylaminobenzyl)-2-imidazoline-4,5-diol,

4(or 5)-ethyl-5(or 4)-methyl-2-(3,4-dimethylphenyl)-2-imidazoline-4,5-diol, and the like.

The mixture is heated rapidly to about 85-95°C, preferably about 90-92°C, stirred thereat for about 1-3 hours, preferably about one hour, and cooled to about 10-30°C, preferably about 20-25°C. The solution is then treated with aqueous alkali hydroxide, or a saturated aqueous solution of an alkali carbonate, while stirring vigorously, to adjust the pH of the solution to about 7-10.5, preferably about 7.3-7.7. Preferably, the pH is adjusted to about 7 with an alkali hydroxide and the final adjustment of the pH is carried out by adding an aqueous solution of an alkali carbonate to promote the crystallization of the resulting precipitate.

The resulting slurry is stirred at about 10-30°C, preferably about 20-25°C, for about 2-18 hours, preferably about 5-10 hours, and the solid is separated therefrom and washed with water. After drying, the "as is" yield is about 65-75%; the purity is about 50-55%.

Purification of the crude product is effected by slurrying it in acetone at about 45-55°C, preferably about 45-50°C, cooling the slurry to about 15-25°C, preferably about 20-25°C, recovering the solid, and washing the same with acetone. The recovery is about 76-87% by weight of the amount of crude charged. The recovered product is then recrystallized from 50% ethanol and water. Several recrystallizations may be necessary depending on the product and degree of purity desired.

The following examples illustrate the present invention. All parts are by weight unless otherwise indicated.

Example 1

Preparation of 4(or 5)-Ethyl-5(or 4)-Methyl-2-Propyl-2-Imidazoline-4,5-Diol Hydrochloride

To a slurry of butyramidine hydrochloride (110.7 grams; 0.90 mole) in dichloromethane (450 mls), stirred at 5°C while cooling in an ice bath, are added water (18 mls), and a saturated aqueous solution of potassium carbonate (0.5 ml). A solution of 2,3-pentanedione (90 grams; 0.90 mole) in dichloromethane (180 mls) is then added to the resulting thin slurry over a period of 18 minutes while maintaining the temperature of the reaction mixture below 5°C.

The resulting mixture is then stirred at 2-10°C for 3 hours, and at 10-19°C for an additional 18 hours. The resulting thick slurry is diluted with acetone (200 mls) and the solid is separated by filtration, washed with acetone, and dried under vacuum. The product, a colorless crystalline solid, weighs 163.5 grams (81.6% of theoretical), and melts at 110-115°C.

Example 2

Preparation of 4(or 5)-(1-Hydroxyethyl)-5(or 4)-Methyl-2-Propylimidazole

A mixture of the product of Example 1 (5.0 grams; 0.022 mole), concentrated hydrochloric acid (4 mls), and water (10 mls) is heated to 90°C, stirred at 90°C for 3 hours, cooled in a ice bath to 15-20°C, and treated with a saturated aqueous solution of potassium carbonate until no further precipitation occurs. The solid is then separated by filtration and dried to obtain 3.1 grams of colorless solid which melts at 175-183°C. Analysis of the nuclear magnetic resonance spectrum of the solid shows that the product contains about 60-70% by weight of the desired product, plus ethers, and the isomeric alcohol 4(5)-ethyl-5(4)-hydroxymethyl-2-propylimidazole.

Two recrystallizations of the crude product from 50% ethanol in water gives the pure compound, m.p. 198-200°C, in a yield about 25% of theoretical.

Example 3

Preparation of Methyl 5(or 4)-Methyl-2-Propyl-4(or 5)-Imidazolyl Ketone

Jones' oxidation reagent (5 mls), prepared by dissolving 1.2 grams of chromium trioxide in 5.14 mls of water, adding 2.13 mls of concentrated sulfuric acid, and diluting with water to 9.2 mls, is added to a slurry of the crude product of Example 2 (1.0 gram) in acetone (25 mls), over a period of 8 minutes at 15-21°C; the reaction mixture is then stirred at ambient temperature for an additional one hour and 15 minutes. The reaction mixture is then treated with isopropanol (6 mls) to decompose excess oxidizing agent, and the resulting liquid layer is separated from a gummy dark solid by decantation. The solvent is evaporated from the liquid layer and the resulting solution is combined with the dark solid. The resulting mixture is treated with a saturated aqueous solution of potassium bicarbonate until alkaline (pH = 8), and then extracted with ethyl acetate (3 times with 75 mls). The ethyl acetate extract is dried over anhydrous sodium sulfate. Comparison of a sample of this material with an ethyl acetate solution of an authentic sample of methyl 5-methyl-2-propyl-4-imidazolyl ketone by thin-layer chromatography shows that they are similar.

Example 4

Preparation of 4(or 5)-Ethyl-5(or 4)-Methyl-2-Phenyl-2-Imidazoline-4,5-Diol Hydrochloride

To a slurry of benzamidine hydrochloride (15.7 grams; 0.1 mole) in dichloromethane (50 mls), stirred at 5°C while cooling in an ice bath, are added water (2 mls), and a

saturated aqueous solution of potassium carbonate (0.05 ml). After stirring for 15 minutes, a solution of 2,3-pentanedione (10 grams; 0.1 mole) in dichloromethane (20 mls) is added thereto over a period of 3 minutes while maintaining the temperature of the reaction mixture below 5°C. The reaction mixture is then stirred at 5°C for 2 hours, and at room temperature (24°C) for an additional 16 hours. The resulting thick slurry is diluted with dichloromethane (25 mls), and acetone (100 mls), stirred for 30 minutes, and filtered to separate the solid. The product, a colorless crystalline solid, weighs 22.0 grams (85.7% of theoretical), and melts at 139-143°C. Recrystallization from water raises the melting point to 140-145°C.

Example 5

Preparation of 4(or 5)-(1-Hydroxyethyl)-5(or 4)-Methyl-2-Phenylimidazole

A mixture of the product of Example 4 (5.0 grams; 0.019 mole), concentrated hydrochloric acid (4 mls), and water (20 mls) is heated to 90°C, stirred at 90°C for one hour, cooled to room temperature (25°C), and treated with 5N sodium hydroxide to adjust the pH to 10. The reaction mixture is stirred for one hour at room temperature and the solid is separated by filtration, washed with water, and dried to obtain 3.15 grams of the desired product, which melts at 198-200°C after recrystallization from 95% ethanol.

## Example 6

**Preparation of 4(or 5)-Methyl-2,5(or 2,4)-di-n-propyl-2-imidazoline-4,5-diol hydrochloride (New Claim B)**

To a slurry of butyramidine hydrochloride (60.8 grams, 0.5 mole) in a mixture of 350 ml of dichloromethane, 10 ml of water and 1 ml of a saturated solution of potassium carbonate, at a temperature below 5°C, was added a solution of 57.1 grams, 0.5 mole, of 2,3-hexanedione in 100 ml of dichloromethane, maintaining a temperature below about 11°C. The resulting mixture was stirred at 5-13°C for about 4 hours and then cooled to 0°C. The reaction mixture was then filtered and the product washed successively with 100 ml of dichloromethane and 130 ml of acetone and dried in vacuo. A colorless crystalline solid (101.8 grams, 86.7% yield) was obtained; m.p. 95-100°C.

## Example 7

**Preparation of 4(or 5)-Methyl-5(or 4)-(1-hydroxypropyl)-2-propylimidazole**

To an aqueous solution of concentrated hydrochloric acid (18.5 grams, 0.19 mole) in 106 ml of water, maintained at 85-90°C, was added the product of Example 6 (23.7 grams, 0.1 mole) over a period of about 10 minutes. The resulting solution was stirred at 80-90°C for about 30 minutes, then cooled to 15-20°C and slowly treated with an aqueous solution of sodium hydroxide (0.15 mole) and then with an aqueous solution of potassium carbonate (0.27 mole) over a period of about 1.5 hours. The solid precipitate was filtered, washed successively with water and acetone (250 ml) and dried. There was obtained 3.2 grams (17.7% yield) of a colorless solid, m.p. 176-177°C, which was found to be a mixture of the desired compound and 4(or 5)-hydroxymethyl-2,5(or 2,4)-di-n- propylimidazole:

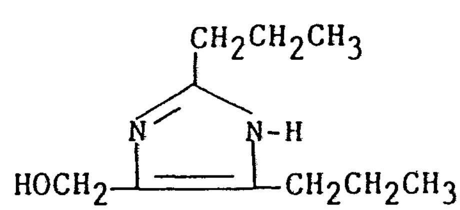

I CLAIM:

1. A process for preparing a compound of the formula

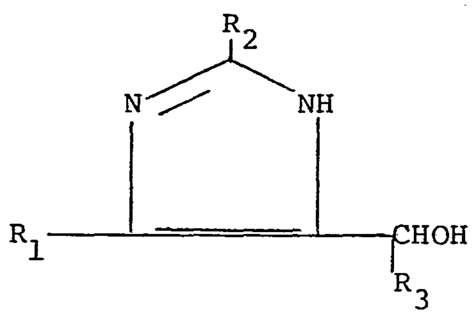

(I)

wherein $R_1$ and $R_3$ are the same or a different straight or branched chain lower alkyl group; and $R_2$ is hydrogen, a straight or branched chain lower alkyl, benzyl, or phenyl group, or a phenyl group substituted with a halo, a straight or branched chain lower alkyl or alkoxy, an amino, a lower dialkylamino or a nitro group, comprising:

adding a compound of the formula

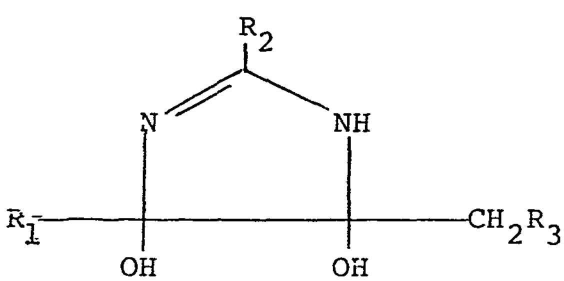

(II)

to an aqueous solution of an acid to form a reaction mixture, the mole ratio of said acid to said formula (II) being about 1.75 to 2.5;

maintaining said reaction mixture at a temperature of about 85 to 95°C for about 1 to 3 hours;

neutralizing said reaction mixture;

recovering the precipitate effected by said neutralizing step; and

separating from said precipitate the compound of formula (I).

2. A process of Claim 1 wherein $R_1$ and $R_3$ are methyl, and $R_2$ is n-propyl.

3. A process of Claim 1 wherein $R_1$ and $R_3$ are

methyl, and $R_2$ is phenyl.

4. A process of Claim 1 wherein $R_1$ is methyl, $R_2$ is propyl and $R_3$ is ethyl.

5. A process of Claim 1 or 2 or 3 or 4 wherein said acid is selected from the group consisting of hydrochloric, sulfuric, phosphoric, boric and p-toluenesulfonic.

6. A process of Claim 5 wherein said acid is hydrochloric acid.

7. A process of Claim 6 wherein the mole ratio of said acid to said formula (II) is about 1.9 to 2.2.

8. A process of Claim 7 wherein said reaction mixture is maintained at about 90 to 92°C for about one hour.

9. The compound 4 (or 5)-methyl-5 (or 4)-ethyl--2-propyl-2-imidazoline-4,5-diol hydrochloride (or 4).

10. The compound 4 (or 5)-methyl-2,5 (or 2,4)--di-n-propyl-2-imidazoline-4,5-diol.

European Patent Office

# EUROPEAN SEARCH REPORT

0106962

Application number

EP 83 10 8038

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | BULLETIN DE LA SOCIETE CHIMIQUE DE FRANCE, no. 3, 1971, pages 1052-1060, Paris, FR. J.-L. IMBACH et al.: "Recherches dans la série des azoles. LXXXII. Condensation d'amidines et de composés alpha-dicarbonylés. Etude des dihydroxy-4,5 imidazolines-2 formées" * Page 1054, column 1, lines 1059-1060 * | 1-10 | C 07 D 233/64 |
| | ----- | | |
| | | | TECHNICAL FIELDS SEARCHED (Int. Cl. 3) |
| | | | C 07 D 233/00 |

The present search report has been drawn up for all claims

| Place of search | Date of completion of the search | Examiner |
|---|---|---|
| THE HAGUE | 30-01-1984 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document